# EUROPEAN PATENT SPECIFICATION

(11) **EP 0 382 220 B1**
(45) Date of publication and mention of the grant of the patent: **28.06.1995**
(21) Application number: 90102504.9
(22) Date of filing: 08.02.1990
(51) Int. Cl.: C07D 277/593

(54) **Method for preparing aminothiazolylacetic acid derivatives**
Verfahren zur Herstellung von Aminothiazolylessigsäure-Derivate
Préparation de dérivés d'acide aminothiazolylacétique

(30) Priority: 10.02.1989 JP 31759/89
(43) Date of publication of application: 16.08.1990
(73) Proprietor: MEIJI SEIKA KAISHA LTD., Chuo-ku Tokyo 104 (JP)
(72) Inventor: Okonogi, Tsuneo, c/o Pharmaceutical Res. Lab., Kohoku-ku, Yokohama-shi, Kanagawa-ken (JP); Murai, Yasushi, c/o Pharmaceutical Res. Lab., Kohoku-ku, Yokohama-shi, Kanagawa-ken (JP); Nishizuka, Toshio, c/o Pharmaceutical Res. Lab., Kohoku-ku, Yokohama-shi, Kanagawa-ken (JP); Arai, Yasuhiko, c/o Pharmaceutical Res. Lab., Kohoku-ku, Yokohama-shi, Kanagawa-ken (JP); Shibahara, Seiji, c/o Pharmaceutical Res. Lab., Kohoku-ku, Yokohama-shi, Kanagawa-ken (JP); Inouye, Shigeharu, c/o Pharmaceutical Res. Lab., Kohoku-ku, Yokohama-shi, Kanagawa-ken (JP)
(74) Representative: Müller-Boré & Partner Patentanwälte

(56) References cited:
- EP-A- 0 099 297
- EP-A- 0 227 953
- FR-A- 2 503 712
- CHEMICAL ABSTRACTS, vol. 104, no. 25, 23rd June 1986, page 615, abstract no.224893x, Columbus, Ohio, US; & JP-A-61 10 569 (SANKEI YAKUHIN K.K.) 18-01-1986

## Description

### BACKGROUND OF THE INVENTION

### Field of the Invention

The present invention relates to new methods for preparing aminothiazolylacetic acid derivatives which are useful as a side chain moiety in the 7-position of cephalosporin derivatives having an antibacterial activity of a broad range, as well as to new methods for preparing intermediates for the derivatives.

### Prior Art

EP-A-0 099 297 shows for instance (tritylamino-2-thiazolyl-4)-2-(tert.-butoxycarbonyl-1-ethyl-1-oxyimino)-2-acetic acid and quite generally the reaction of 2-(tritylamino-4-thiazolyl)-2-(2-tert.-butoxycarbonyl-2-propyloxyimino)-2-acetic acid with cephalosporine derivatives.

The same is shown by FR-A-2 503 712. EP-A-0 227 953 of partly the same inventors as in the present invention also shows reaction of 2-(2-tritylamino-4-thiazolyl)-2-(1S)-(1-diphenylmethoxycarbonyl)-ethoxyimino acetic acid or the corresponding 1R compound with cephalosporines.

The present inventors previously found that compounds having an aminothiazolylacetic acid group of a formula: 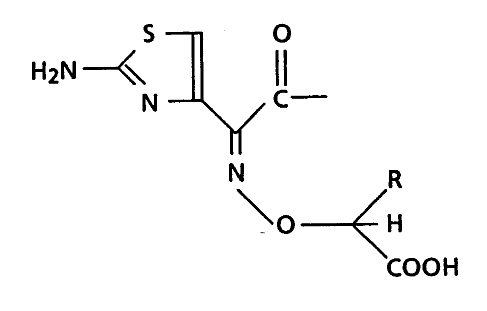
wherein R means an alkyl group, in the side chain of the 7-position of cephalosporin derivatives are useful as antibiotics having an antibacterial activity of a broad range.

They disclosed various methods for preparing aminothiazolylacetic acid group-having compounds, for example, those mentioned below.
(1) An (α-hydroxyimino)aminothiazolylacetic acid derivative (i) is reacted with an α-haloacetic acid having an alkyl group R (ii) in the presence of a strong base to obtain a compound having the group R in the alkoxime moiety (iii); and the resulting compound (iii) is subjected to optical resolution to obtain an optical active isomer thereof (refer to the following reaction formula). 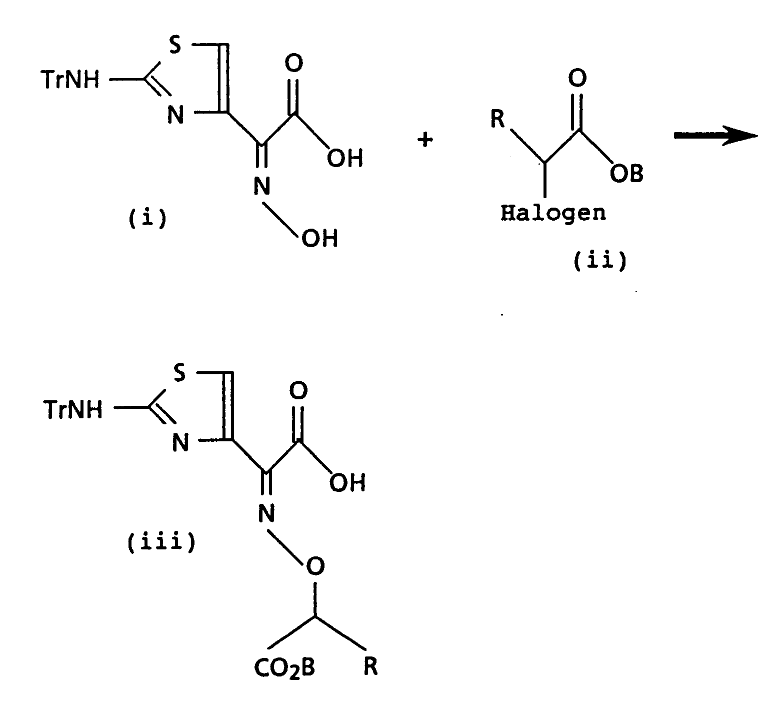 wherein Tr represents a triphenylmethyl group, and B represents a protective group for the carboxylic acid.
(2) An optical active α-hydroxy acid (iv) is reacted with an N-hydroxyphthalimide by dehydration in the presence of triphenylphosphine and diethyl azodicarboxylate to give an N-alkoxyphthalimide derivative (v), this is treated with hydrazine to give an O-alkylhydroxyamine derivative (vi), and the compound is then reacted with an (α-oxo)aminothiazolylacetic acid derivative (vii) to obtain an optical active compound (iii) (refer to the following reaction formula). 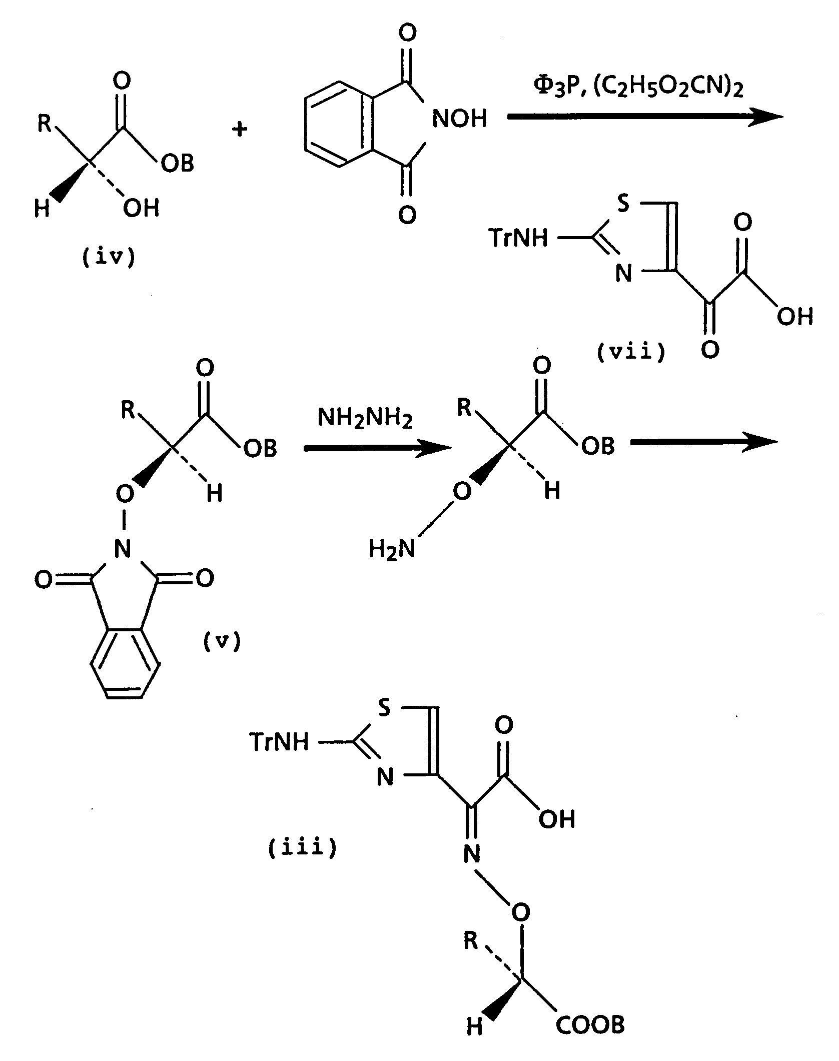 wherein Tr, R and B have the same meanings as mentioned above.
(3) An optical active α-hydroxy acid (iv) is treated with toluenesulfonyl chloride or methanesulfonyl chloride in the presence of a base to give a toluenesulfonate or methanesulfonate (viii), and the compound is then reacted with an (α-hydroxyimino)aminothiazolylacetic acid derivative (ix) in the presence of a base to obtain an optical active compound (iii) (refer to the following reaction formula and Japanese Patent Laid-open Application (KOKAI) No. 62-126189). 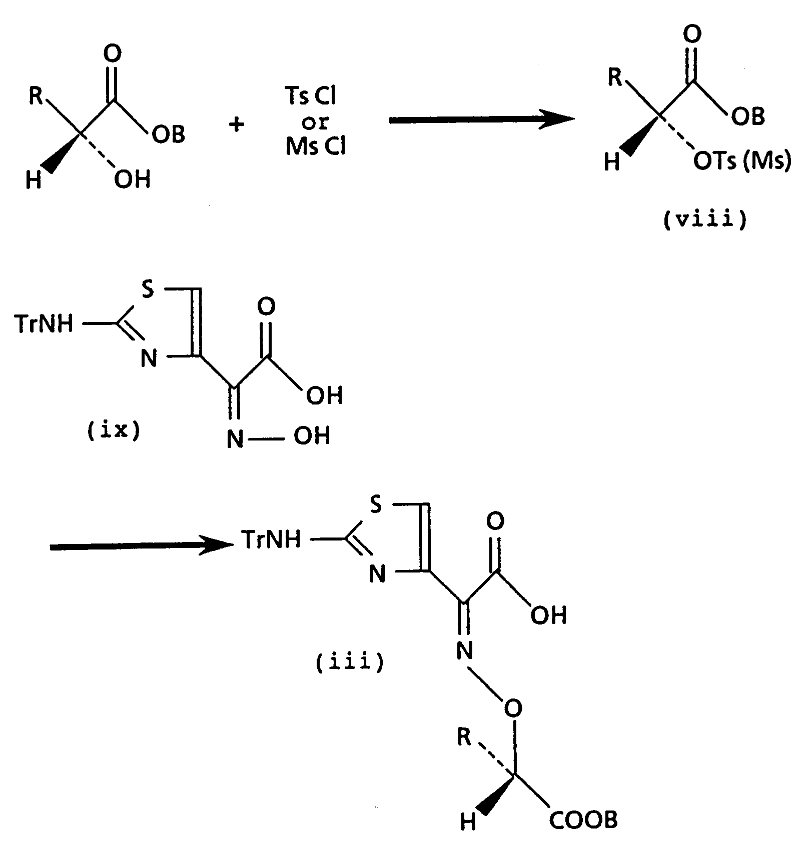 wherein Ms represents a methanesulfonyl group, Ts represents a toluenesulfonyl group, and R and B have the same meanings as mentioned above.

As mentioned above, the cephalosporin derivatives having an aminothiazolylacetic acid group in the side chain of the 7-position, which have an antibacterial activity of a broad range, include the optical active form and the racemic form, as the aminothiazolylacetic group in the 7-position contains an asymmetric carbon atom. Among the two types, the optical active cephalosporin derivatives have a higher antibacterial activity than the racemic cephalosporin derivatives. Accordingly, in the conventional methods, the aminothiazolylacetic acid derivatives obtained by synthesis are subjected to optical resolution or, alternatively, optical active compounds are employed as the starting material for producing optical active products. However, the conventional methods have some drawbacks. Specifically, the former requires the optical resolution step and the yield of the product is low. The latter often produces racemic compounds during reaction, and the yield of the desired optical active product is low.

### SUMMARY OF THE INVENTION

one object of the present invention is to stereo-specifically and industrially produce aminothiazolylacetic acid derivatives.

Another object of the present invention is to provide compounds which are easily bonded to cephalosporin nuclei as well as intermediates for such compounds.

In order to attain the said objects, the present inventors earnestly studied and have found that reaction of an allyl aminothiazole-acetate and a reactive derivative of an optical active propionic acid on the α-position of the former ester gives a new compound allyl (Z)-2-(2-tritylaminothiazol-4-yl)-2-[[(1S) or (1R)-1-(diphenylmethoxycarbonyl)ethoxy]imino]acetate of high optical purity without racemization.

They have further found that deallylation of the resulting compound gives a compound of a free carboxylic acid or an alkali metal salt or quaternary ammonium salt thereof, which is easily reactable with cephem nuclei. on the basis of such findings, they have hereby achieved the present invention.

Specifically, the present invention provides new methods for preparing compounds of a general formula (I): 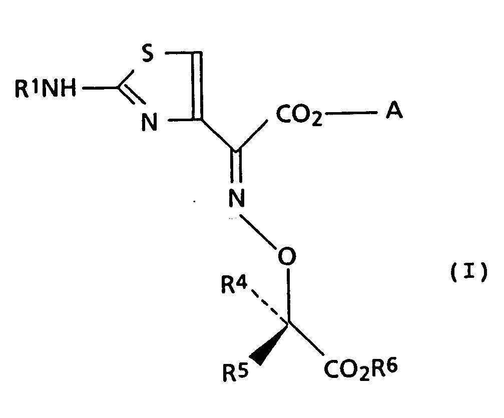
wherein R¹ represents an easily cleavable amino-protective group;
R⁴ and R⁵ are different from each other and each represents a hydrogen atom or a methyl group;
R⁶ represents a carboxylic acid-protective group; and
A represents a hydrogen atom, an alkali metal or a quaternary ammonium salt, wherein a compound of a general formula (II): 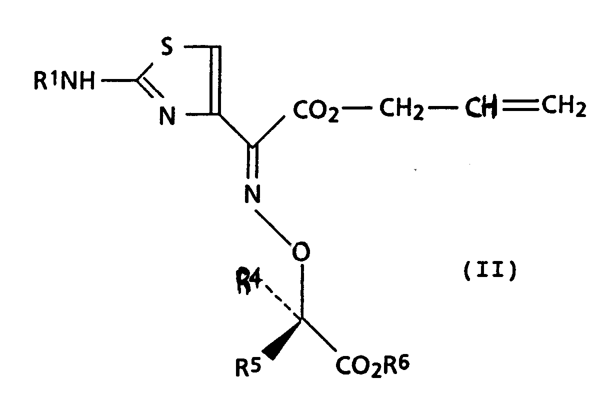
wherein R¹, R⁴, R⁵ and R⁶ have the same meanings as mentioned above.
is reacted with a palladium salt or a palladium compound in the presence of a triphenylphosphine or trialkyl phosphite and an alkali metal alkanoate or an organic base.

As another embodiment of the present invention, there is also provided a method for producing compounds of a general formula (II): 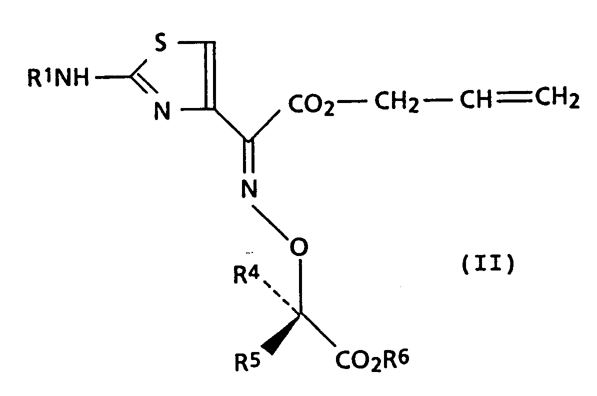
wherein R¹, R⁴, R⁵ and R⁶ have the same meanings as mentioned above, in which a compound of a general formula (III): 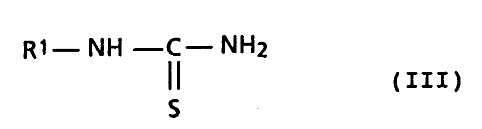
wherein R¹ has the same meaning as mentioned above, is reacted with a compound of a general formula (IV): 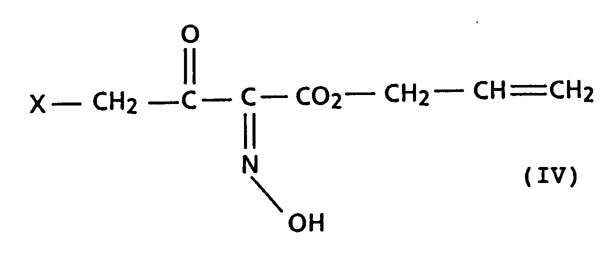
wherein X represents a leaving group, to give a compound of a general formula (V): 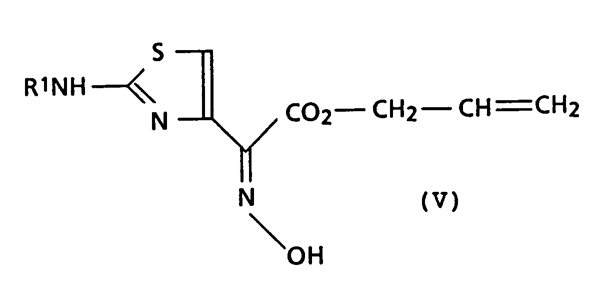
wherein R¹ has the same meaning as mentioned above, and thereafter the compound is reacted with a compound of a general formula (VI): 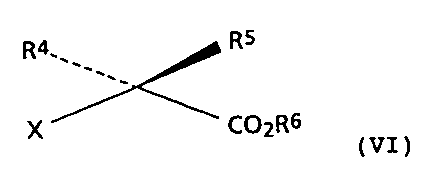
wherein X, R⁴, R⁵ and R⁶ have the same meanings as mentioned above, to obtain the compound of the formula (II).

As still another embodiment of the present invention, there is further provided a method for producing compounds of a general formula (II): 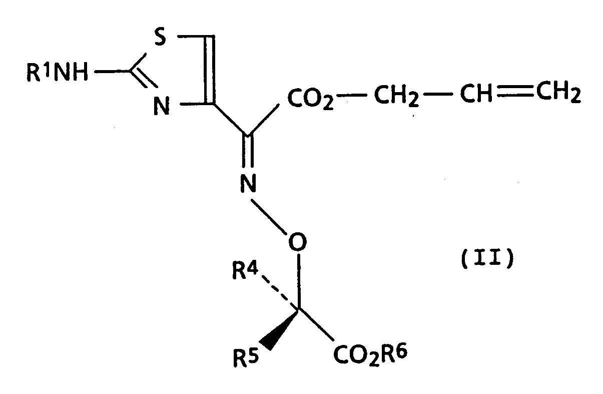
wherein R¹, R⁴, R⁵ and R⁶ have the same meanings as mentioned above, in which a compound of a general formula (VII): 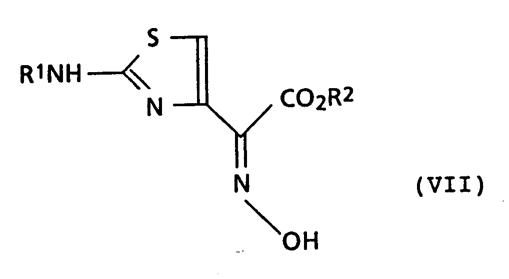
wherein R² represents a lower alkyl group, and R¹ has the same meaning as mentioned above, is reacted with a compound of a general formula (VIII):

R³―O⁻M⁺ (VIII)

wherein R³ represents a lower alkyl group, having preferably 1 to 5 carbon atoms, and M represents an alkali metal, in an allyl alcohol to give a compound of a general formula (V): 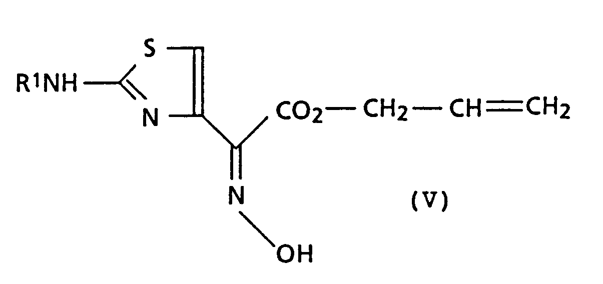
wherein R¹ has the same meaning as mentioned above, and then the compound is reacted with a compound of a general formula (VI): 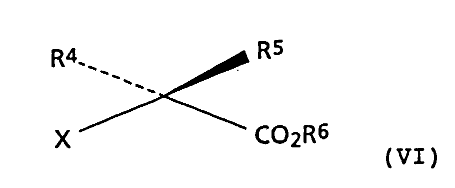
wherein R⁴, R⁵, R⁶ and X have the same meanings as mentioned above, to obtain the compound of the formula (II).

### DETAILED DESCRIPTION OF THE INVENTION

In the compounds of the formula (I) of the present invention, R¹ is an easily cleavable amino-protective group which is generally employed in chemical synthesis in this technical field, and it includes, for example, trityl group, formyl group, acetyl group, t-butoxycarbonyl group, 2,2,2-trichloroethoxycarbonyl group, benzyloxycarbonyl group and p-methoxybenzyloxycarbonyl group.
R⁶ in the same formula is a carboxylic acid-protective group which is generally employed in the field of penicillins and cephalosporins, and it includes, for example, methyl group, ethyl group, t-butyl group, 2,2,2-trichloroethyl group, 2-trimethylsilylethyl group, benzyl group, p-nitrobenzyl group, p-methoxybenzyl group and diphenylmethyl group.

A in the formula (I) is a hydrogen atom, an alkali metal such as sodium or potassium, or a quaternary ammonium group derived from allylation of an organic base such as triethylamine, N-methylmorpholine, N-methylpyrrolidine or N-methylpiperidine.

Specific examples of the compounds of the formula (I) are mentioned below, which, however, are not limitative.
(Z)-2-(2-tritylaminothiazol-4-yl)-2-[[(1S)-1-(diphenylmethoxycarbonyl)ethoxy]imino]acetic acid
Potassium (Z)-2-(2-tritylaminothiazol-4-yl)-2-[[(1S)-1-(diphenylmethoxycarbonyl)ethoxy]imino]acetate
(Z)-2-(2-tritylaminothiazol-4-yl)-2-[[(1R)-1-(diphenylmethoxycarbonyl)ethoxy]imino]acetic acid
N-allyl-N-methylpyrrolidinium (Z)-2-(2-tritylaminothiazol-4-yl)-2-[[(1S)-1-(diphenylmethoxycarbonyl)ethoxy]imino]acetate
R¹ and R⁶ of the compounds of the formula (II) of the present invention may be the same protective groups as those in the compounds of the formula (I).

Specific examples of the compounds of the formula (II) are mentioned below, which also are not limitative.
Allyl (Z)-2-(2-tritylaminothiazol-4-yl)-2-[[(1S)-1-(diphenylmethoxycarbonyl)ethoxy]imino]acetate
Allyl (Z)-2-(2-tritylaminothiazol-4-yl)-2-[[(1R)-1-(diphenylmethoxycarbonyl)ethoxy]imino]acetate
The first method of the present invention to prepare the compounds of the formula (II) comprises the first step of reacting the compound of the formula (III) and the compound of the formula (IV) to produce the compound of the formula (V) and the second step of reacting the compound of the formula (V) as prepared in the first step and the compound of the formula (VI) to produce the compound of the formula (II). The reaction procedure may be represented by the following reaction formulae. 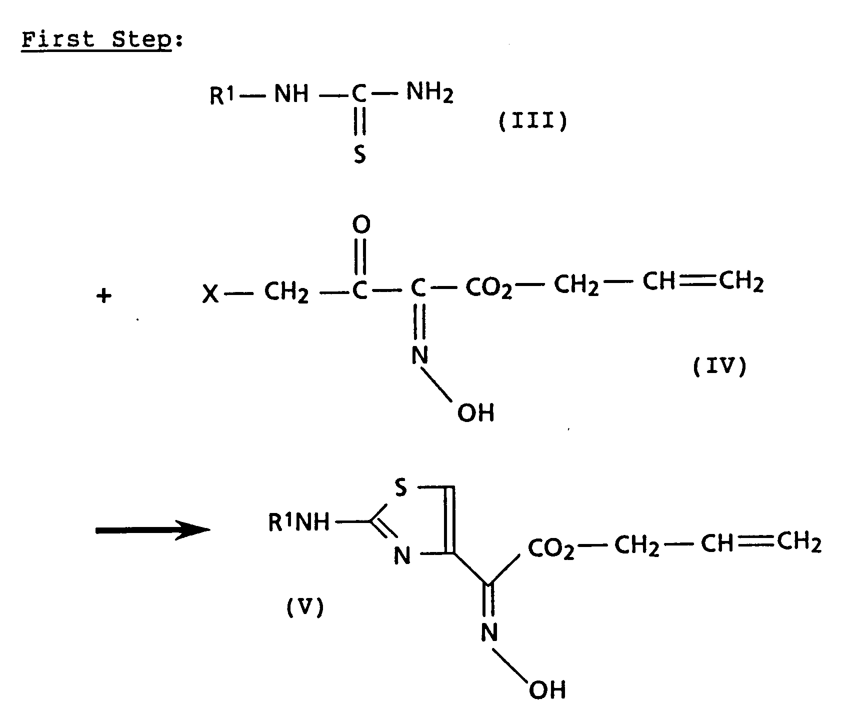
wherein R¹ and X have the same meanings as mentioned above.

The amino-protective group of R¹ in the thiourea derivatives of the formula (III) may be the same as that in the compounds of the above-mentioned formula (I).

The leaving group of X in the allyl 3-oxo-2-(hydroxyimino)butyrate derivatives of the formula (IV) includes, for example, a halogen atom such as a chlorine, bromine or iodine atom, or a methanesulfonyloxy group, trifluoromethanesulfonyloxy group or toluenesulfonyloxy group.

The reaction is generally conducted in an organic solvent or in a water-containing organic solvent, in the presence of a base at a temperature of a broad range.

The base usable in the reaction includes, for example, organic bases such as pyridine or N,N-dimethylaniline as well as salts such as sodium acetate or potassium acetate. The usable solvent includes, for example, N,N-dimethylformamide, N,N-dimethylacetamide, dimethyl sulfoxide, chloroform, methylene chloride, ethyl acetate, acetone, acetonitrile, dioxane, methanol and ethanol.

The compound of the formula (V) to be obtained by the reaction is, in general, directly used in the next second step without being isolated from the reaction solution. 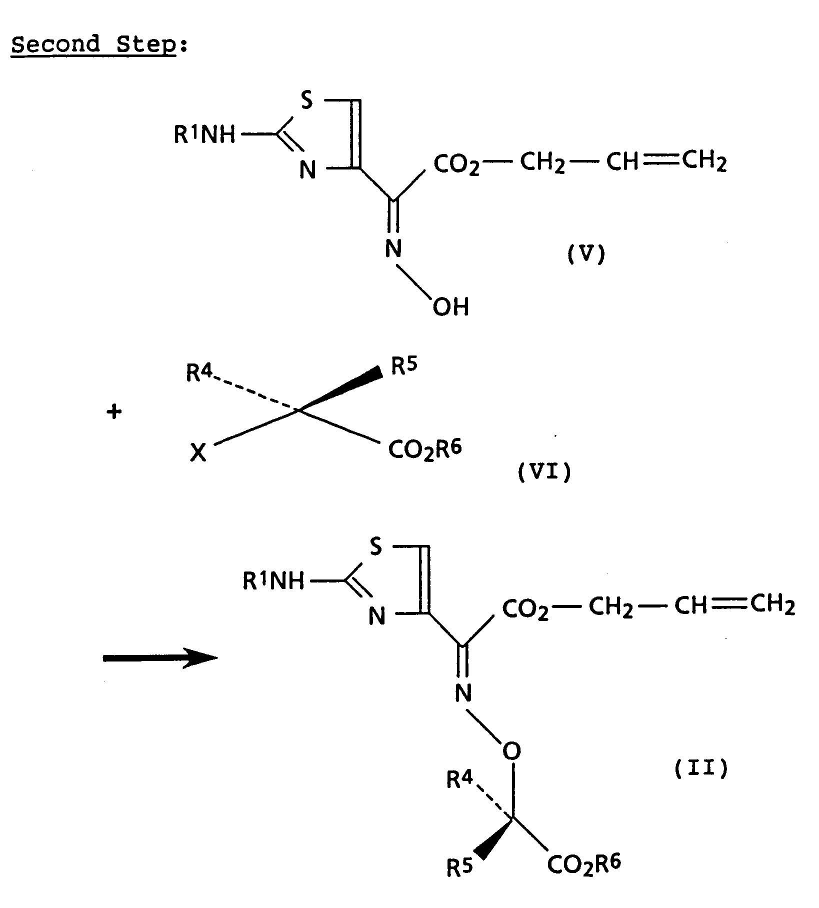
wherein R¹, R⁴, R⁵, R⁶ and X have the same meanings as mentioned above.

In the propionate derivatives of the formula (VI) to be employed in the reaction step, R⁵ is methyl group when R⁴ is hydrogen atom; and R⁵ is hydrogen atom when R⁴ is methyl group. The leaving group X in the compounds of the formula (VI) may be the same as that in the compounds of the formula (IV) employed in the previous first step.

The propionate derivatives of the formula (VI) which are employed in the present reaction step are to be optical active ones. These can be produced from optical active (S)-lactic acid esters (1) in the form of the desired enantiomers, in accordance with the following reaction formula: 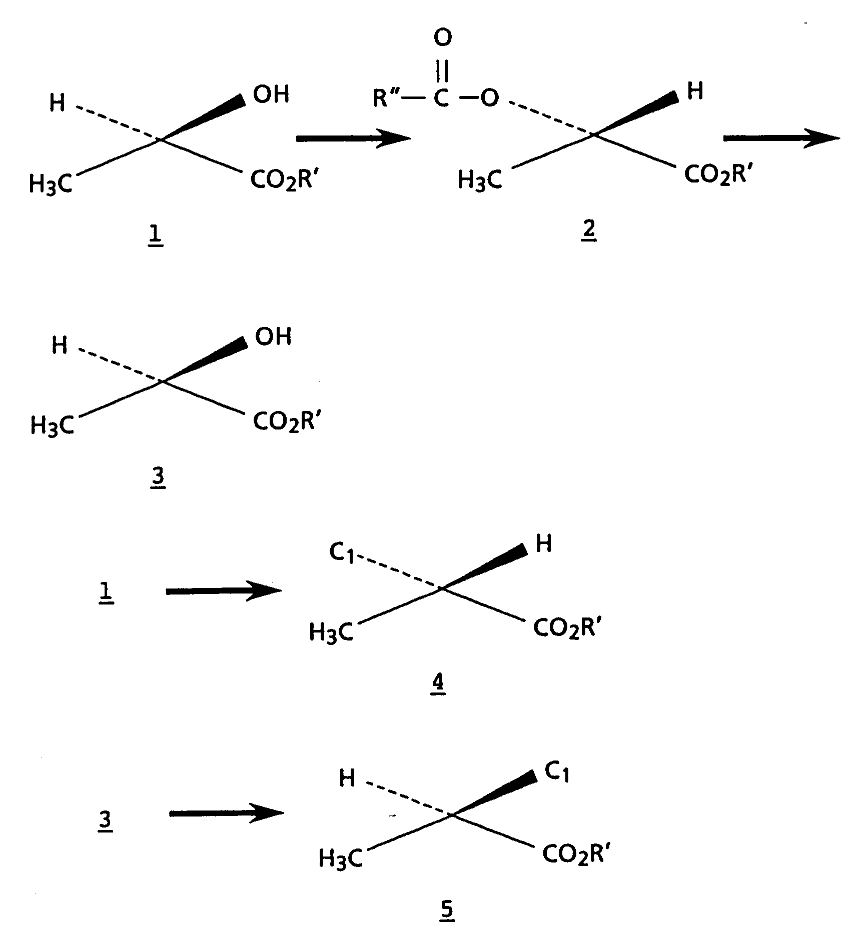
wherein R' represents a carboxylic acid-protective group; and R'' represents a hydrogen atom or a phenoxymethyl group.

Where an (S)-lactic acid ester (1) which can be produced from an optical active and low-priced lactic acid is reacted with diethyl azodicarboxylate in the presence of formic acid or phenoxyacetic acid and triphenylphosphine, an (R)-lactic acid ester derivative (2) is produced by α-carbon inversion.

The resulting compound is hydrolyzed in a conventional manner to give an (R)-lactic acid ester (3) with a high yield.

Each of the (S)-lactic acid ester (1) and (R)-lactic acid ester (3) is treated with sulfuryl chloride in a solvent of dimethylformamide at a low temperature, whereby an (R)-2-chloropropionic acid ester (4) is derived from the (S)-lactic acid ester (1) and an (S)-2-chloropropionate (5) from the (R)-lactic acid ester (3), respectively, by chlorination followed by inversion.

The reaction of the second step is generally conducted in a solvent such as N,N-dimethylformamide or dimethylsulfoxide, in the presence of a base such as anhydrous potassium carbonate, anhydrous sodium carbonate, potassium hydrogencarbonate or sodium hydrogencarbonate, at a temperature falling within the range of from 0 to 60°C, preferably at approximately 40°C.

The second method of the present invention to prepare the compounds of the formula (II) comprises the first step of reacting the compound of the formula (VII) and the compound of the formula (VIII) in allyl alcohol to produce the compound of the formula (V) and the second step of reacting the compound of the formula (V) and the compound of the formula (VI) to produce the compound of the formula (II). The reaction procedure may be represented by the following reaction formulae: 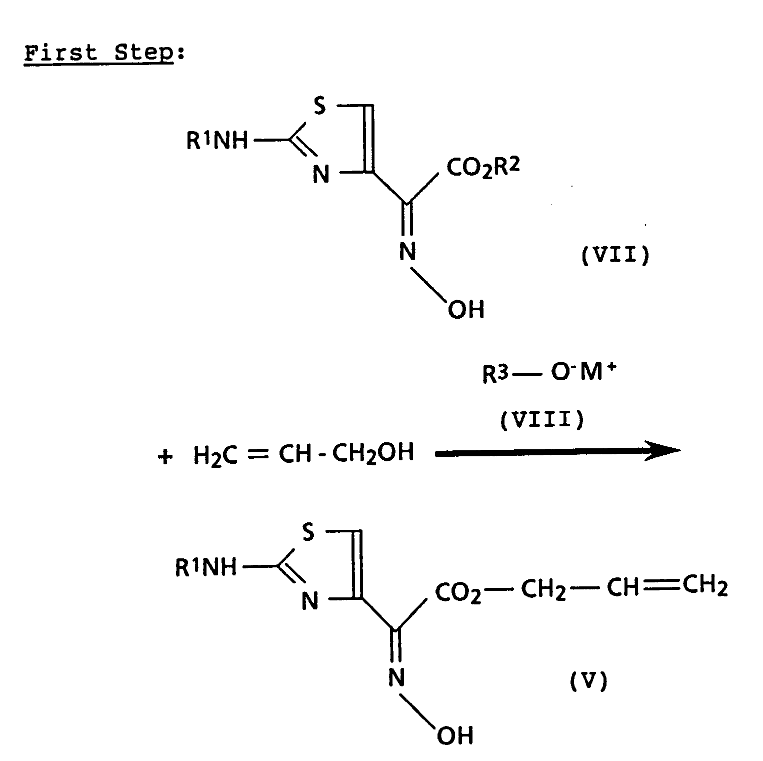
wherein R² and R³ each represent a lower alkyl group, having preferably 1 to 5 carbon atoms, M represents an alkali metal and R¹ has the same meaning as mentioned above.

The amino-protective group of R¹ in the compounds of the formula (VII) may be the same as that in the compounds of the above-mentioned formula (I). R² in the formula (VII) is a carboxylic acid-protective group, and it includes, for example, a lower alkyl group such as methyl group, ethyl group, n-propyl group, isobutyl group or n-butyl group.

The compounds of the formula (VIII) are alkali metal alkoxides, which include, for example, sodium methoxide, sodium ethoxide, sodium t-butoxide, potassium methoxide, potassium t-butoxide or lithium methoxide.

The reaction is generally conducted at a temperature falling within the range of from room temperature to 100°C, using a solvent amount (generally, from 8 to 20 times amount) of allyl alcohol and a catalytic amount (generally, from 1/50 to 1/5 equivalent amount) of the alkali metal alkoxide of the formula (VIII). Preferably, 12 times amount of allyl alcohol and 3/100 equivalent amount of sodium ethoxide are used and the reaction is effected at 60°C and may be finished in about 4 hours.

The compound of the formula (V) to be obtained by the reaction is used in the second step.

The second step of the second method is same as the second step of the above-mentioned first method.

The compounds of the formula (II) prepared by the above-mentioned methods are new compounds and are useful as intermediates for producing the compounds of the formula (I) which can be introduced into the side chain of the 7-position of cephalosporin derivatives having an antibacterial activity of a broad range.

For producing the compounds of the formula (I) of the present invention, the allyl group is cleaved out from the compound of the formula (II) as prepared by the above-mentioned methods and accordingly, the compound of the formula (I) is prepared in the form of an alkali metal salt or quaternary ammonium salt of the carboxylic acid or in the form of a free carboxylic acid of itself. The reaction procedure can be represented by the following reaction formula: 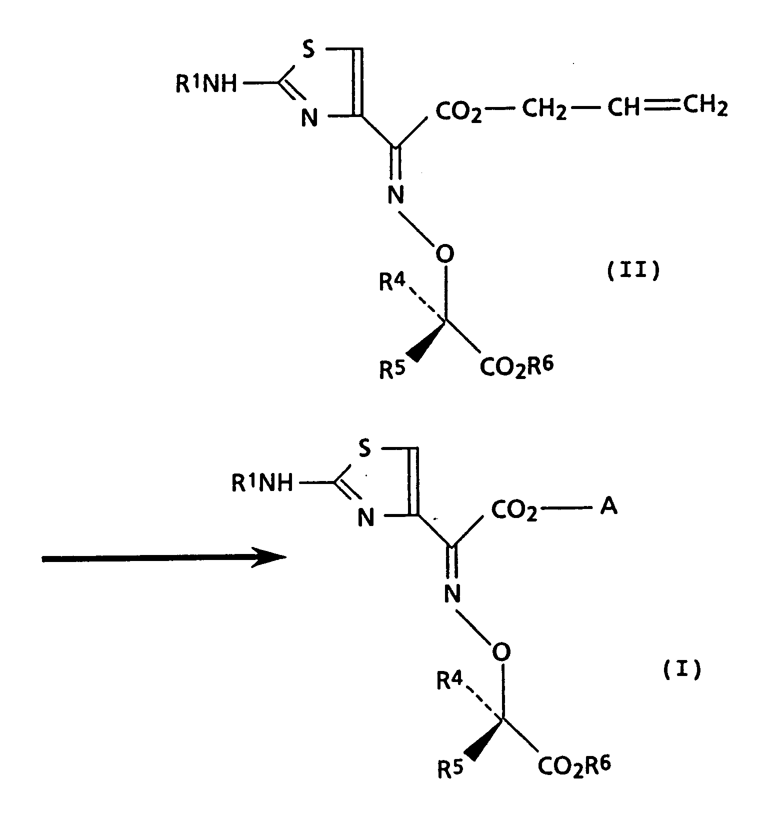
wherein R¹, R⁴, R⁵, R⁶ and A have the same meanings as mentioned above.

In the reaction, the compound of the formula (II) is reacted with a complex of a triphenylphosphine or trialkyl phosphite and a palladium metal to be formed from a triphenylphosphine or trialkyl phosphite and a palladium salt, or with a tetrakis(triphenylphosphine) palladium, in the presence of an alkali metal alkanoate such as sodium acetate, sodium 2-ethylhexanoate or potassium 2-ethylhexanoate or an organic base such as triethylamine, N-methylmorpholine or N-methylpiperidine. The reaction is generally conducted in an inert solvent such as ethyl acetate or methylene chloride, at a temperature of 0°C to 100°C.

The compounds of the formula (I) to be formed by the reaction are isolated in the form of alkali metal salts where an alkali metal alkanoate is in the reaction solution, while they are isolated in the form of allylated quaternary ammonium salts where an organic base is therein. After the salts are treated with an acid, the products are isolated in the form of free acids.

The compounds of the formula (I) prepared as mentioned above are advantageously introduced into the side chain of the 7-position of cephalosporin derivatives having an antibacterial activity of a broad range.

The present invention will be explained in more detail by way of the following examples which illustrate production of the new compounds of the present invention as well as intermediates thereof. However, the present invention is not whatsoever restricted by the following examples. In addition, production of propionate derivatives of the formula (VI) is also illustrated by referential examples.

### EXAMPLE 1:

### Allyl (Z)-2-(2-tritylaminothiazol-4-yl)-2-(hydroxyimino)acetate:

10 g of N-tritylthiourea and 7.15 g of allyl 4-chloro-3-oxo-2-(hydroxyimino)butyrate were dissolved in 50 ml of dimethylformamide, and 2.75 ml of pyridine was added thereto and stirred for 4 hours at 30°C.

The reaction solution is poured into a mixture comprising 150 ml of ethyl acetate and 150 ml of water. After the pH value of the resulting mixture was adjusted to 2.0, the organic layer was separated by liquid-liquid separation.

The organic layer was washed with water and dried, and then the solvent was removed by distillation. The resulting residue was crystallized from butyl acetate to obtain 6.89 g of the above-entitled product.
m.p.: 187 - 189°C
NMR(CDCl₃) δ :
4.73(2H,m), 5.15(1H,m), 5.33(1H,m), 5.90(1H,s), 7.10 - 7.40(15H,m)

### EXAMPLE 2:

### Allyl (Z)-2-(2-benzyloxycarbonylaminothiazol-4-yl)-2-(hydroxyimino)acetate:

The same process as in Example 1 was repeated except that the N-tritylthiourea was replaced by n-benzyloxycarbonylthiourea, and the above-entitled compound was obtained.
NMR(DMSO-d₆) δ :
4.75(2H,m), 5.22(2H,s), 5.30(2H,m), 5.88(1H,m), 7.36(5H,s), 7.42(1H,s), 11.8(1H,s), 12.0(1H,s)

### EXAMPLE 3:

### Allyl (Z)-2-[2-(2,2,2-trichloroethoxycarbonylamino)thiazol-4-yl]-2-(hydroxyimino)acetate:

The same process as in Example 1 was repeated except that the N-tritylthiourea was replaced by N-(2,2,2-trichloroethoxycarbonyl)thiourea, and the above-entitled product was obtained.
NMR(DMSO-d₆) δ :
4.77(2H,m), 4.97(2H,s), 5.30(2H,m), 5.90(1H,m), 7.48(1H,s), 11.9(1H,s), 12.0(1H,s)

### EXAMPLE 4:

### Allyl (Z)-2-(2-tritylaminothiazol-4-yl)-2-(hydroxyimino)acetate:

64 g of ethyl (Z)-2-(2-tritylaminothiazol-4-yl)-2-(hydroxyimino)acetate was added to 770 ml of allyl alcohol and heated up to 60°C. 0.33 g of sodium ethoxide was added to the resulting solution and stirred for 4 hours at the same temperature, and then the reaction solution was concentrated.

200 ml of toluene was added to the resulting residue, which was again concentrated. The crude product thus precipitated was taken out by filtration. This was dissolved in 380 ml of methylene chloride, washed with water and concentrated, and 200 ml of ethyl acetate was added thereto. This was again concentrated.

The crystal thus precipitated was taken out by filtration, washed with ethyl acetate and then dried at 40°C under reduced pressure to obtain 51 g of the above-entitled product.

### EXAMPLE 5:

### (Z)-2-(2-tritylaminothiazol-4-yl)-2-[[(1S)-1-(diphenylmethoxycarbonyl)ethoxy]imino]acetic Acid:

### (a):

50 g of allyl (Z)-2-(2-tritylaminothiazol-4-yl)-2-(hydroxyimino)acetate was dissolved in 260 ml of dimethyl sulfoxide, and 32 g of diphenylmethyl (R)-chloropropionate and 29 g of powdery anhydrous potassium carbonate were added thereto and stirred for 4 hours at 40°C.

The reaction solution was poured into a mixture comprising 400 ml of ethyl acetate and 400 ml of water, and the organic layer was separated by liquid-liquid separation. The resulting organic layer was washed with 10 % NaCl solution and concentrated to obtain allyl (Z)-2-(2-tritylaminothiazol-4-yl)-2-[[(1S)-1-(diphenylmethoxycarbonyl)ethoxy]imino]acetate as an oily product.

The compound was directly used in the next reaction without being isolated and purified further. For reference, the physical properties of the purified compound are shown below.
NMR(CDCl₃) δ :
1.50(3H,d,J=7.03Hz), 4.75(2H,m), 4.93(1H,q,J=7.03Hz), 5.25(2H,m), 5.80(1H,m), 6.51(1H,s), 6.89(1H,s), 7.10 - 7.50(25H,m)

### (b):

The compound prepared above was dissolved in 250 ml of ethyl acetate, and methylene chloride solution containing 0.27 g of tetrakis(triphenylphosphine) palladium was added thereto in nitrogen stream atmosphere and stirred for 30 minutes at room temperature. Next, 87 ml of ethyl acetate solution containing 19.2 g of potassium 2-ethylhexanoate was added thereto and stirred for 1 hour.

500 ml of ethyl alcohol was added to the reaction solution and stirred overnight at 5°C, and the crystal as precipitated was taken out by filtration. Accordingly, potassium (Z)-2-(2-tritylaminothiazol-4-yl)-2-[[(1S)-1-(diphenylmethoxycarbonyl)ethoxy]imino] acetate was obtained.

The compound was dissolved in 500 ml of methylene chloride, 250 ml of water was added thereto, and the resulting solution was adjusted to pH of 2.0 with 2N hydrochloric acid with cooling with ice. Afterwards, the solution was subjected to liquid-liquid separation.

The organic layer thus separated was taken out, washed with water and then concentrated to 350 ml. Next, 700 ml of n-hexane was added thereto and stirred overnight. The crystal thus precipitate was taken out by filtration to obtain 53 g of (Z)-2-(2-tritylaminothiazol-4-yl)-2-[[(1S)-1-(diphenylmethoxycarbonyl) ethoxy]imino]acetic acid.
m.p.: 135 - 137°C (decomposition)
[α]_{D} : -10.2 (C5.0, CHCl₃)
MNR (CDCl₃) δ :
1.50(3H,d,J=7.03Hz), 5.05(1H,q,J=7.03Hz), 6.62(1H,s), 6.90(1H,s), 7.00 - 7.40(26H,m)

### EXAMPLE 6:

### (Z)-2-(2-tritylaminothiazol-4-yl)-2-[[(1R)-1-diphenylmethoxycarbonyl)ethoxy]imino]acetic Acid:

The same process as in Example 5 was repeated except that the diphenylmethyl (R)-chloropropionate was replaced by the corresponding optical isomer (S)-chloropropionate, and the above-entitled product was obtained.
[α]_{D} : +9.80 (C5.0, CHCl₃)
NMR(CDCl₃) δ :
1.53(3H,d,J=7.05Hz), 5.07(1H,q,J=7.05Hz), 6.60(1H,s), 6.93(1H,s), 7.00 - 7.40(26H,m)

### REFERENTIAL EXAMPLE 1:

### Diphenylmethyl (R)-2-hydroxypropionate:

8.0 g of diphenylmethyl (S)-2-hydroxypropionate, 4.9 g of phenoxyacetic acid and 9.8 g of triphenylphosphine were dissolved in 200 ml of tetrahydrofuran, and 7.6 g of diethyl azodicarboxylate was added thereto with cooling with ice and thereafter stirred for 15 hours.

The reaction solution was concentrated and the residue was dissolved in 200 ml of n-hexane, whereupon crystals of triphenylphosphine oxide precipitated out. The crystals were isolated by filtration, and the resulting filtrate was washed with an acidic water having a pH value of 3.0, an aqueous sodium hydrogencarbonate solution and water in this order and then concentrated. Accordingly, an oily product of diphenylmethyl (R)-2-(phenoxyacetoxy)propionate was obtained. The product was used in the next reaction without being further purified. For reference, the physical properties of the purified product are shown below.
[α]_{D} : +29 (C5.0, CHCl₃)
NMR(CDCl₃) δ :
1.47(3H,d,J=6.81Hz), 4.62(2H,s), 5.30(1H,q,J=6.81Hz), 6.10 - 7.40(16H,m)
The above-mentioned product was dissolved in 100 ml of methanol, and 1.4 ml of 25 % aqueous ammonia was added thereto and stirred for 1 hour at room temperature. The reaction solution was adjusted to have a pH value of 7.0 and then concentrated, and the residue was dissolved in ethyl acetate and then washed with water. The resulting extract was dried and concentrated, and the residue was crystallized out from isopropyl ether to obtain 5.6 g of the above-entitled product.
m.p.: 80 - 82°C
[α]_{D} : +9.1 (C5.0, CHCl₃)
NMR(CDCl₃) δ :
1.46(3H,d,J=6.81Hz), 2.78(1H,d,J=5.28Hz), 4.36(1H,dq,J=5.28,6.81Hz), 6.93(1H,s), 7.20 - 7.40(10H,m)

### REFERENTIAL EXAMPLE 2:

### Diphenylmethyl (R)-2-Chloropropionate:

4.0 g of diphenylmethyl (S)-2-hydroxypropionate was dissolved in 20 ml of dimethylformamide, and 2.53 g of sulfuryl chloride was dropwise added thereto at -50°C. Then the whole was stirred for 30 minutes at -50 to -40°C and thereafter for 1 hour at -40 to 25°C.

The reaction solution was poured into a mixture comprising 40 ml of ethyl acetate and 40 ml of water, which contained 5.9 g of sodium hydrogencarbonate, and stirred for 30 minutes and then subjected to liquid-liquid separation.

The resulting extract was washed with a saturated salt solution and water in that order, dried and concentrated. The residue was crystallized from isopropyl alcohol to obtain 3.3 g of the above-entitled product.
m.p.: 57 - 59°C
[α]_{D} : +7.8 (C5.0, CHCl₃)
NMR(CDCl₃) δ :
1.70(3H,d,J=7.03Hz), 4.48(1H,q,J=7.03Hz), 6.89(1H,s), 7.20 - 7.40(10H,m)

### REFERENTIAL EXAMPLE 3:

### Diphenylmethyl (S)-2-Chloropropionate:

The same process as in Referential Example 2 was repeated except that the diphenylmethyl (S)-2-hydroxypropionate was replaced by diphenylmethyl (R)-2-hydroxypropionate, and the above-entitled product was obtained.
m.p.: 57 - 59°C
[α]_{D} : -7.6 (C5.0, CHCl₃)
As mentioned in detail in the above, the present invention provides the new compounds of the formula (I) which may be the constitutional moiety of the side chain of the 7-position of cephalosporin derivatives having an antibacterial activity of a broad range. In accordance with the present invention, the new compounds of the formula (I) can be produced industrially advantageously from the new intermediates of the formula (II), and the compounds of the formula (I) obtainable by the method of the present invention have a high optical purity.

## Claims

1. A method for preparing compounds of a general formula: 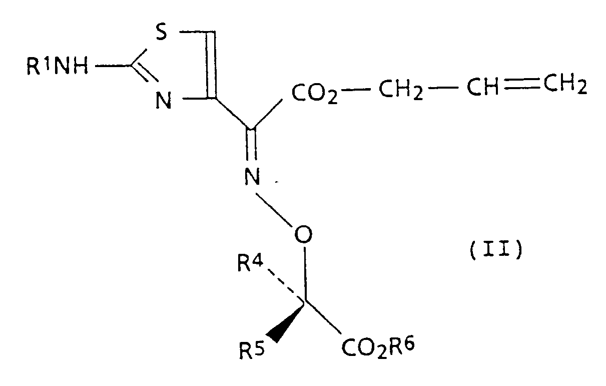 wherein R¹ represents an easily cleavable amino-protective group;
R⁴ and R⁵ are different from each other and each represents a hydrogen atom or a methyl group; and R⁶ represents a carboxylic acid-protective group;
wherein a compound of a general formula: 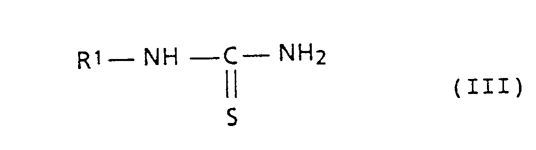 wherein R¹ has the same meaning as mentioned above, is reacted with a compound of a general formula: 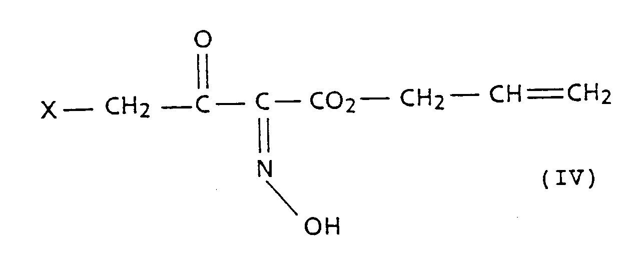 wherein X represents a leaving group, to give a compound of a general formula: 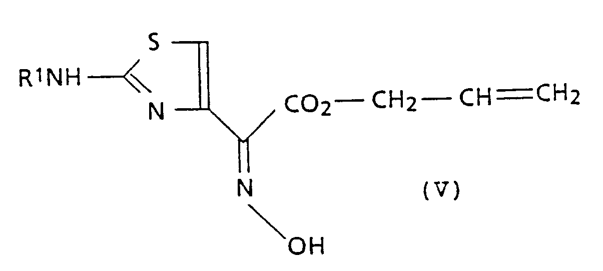 wherein R¹ has the same meaning as mentioned above, and thereafter the resulting compound is reacted with a compound of a general formula: 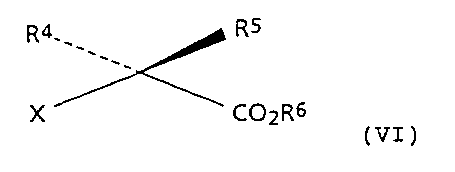 wherein R⁴, R⁵, R⁶ and X have the same meanings as mentioned above.

2. A method for preparing compounds of a general formula: 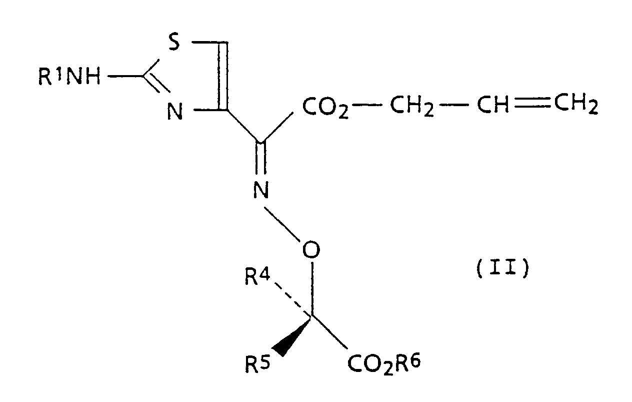 wherein R¹ represents an easily cleavable amino-protective group;
R⁴ and R⁵ are different from each other and each represents a hydrogen atom or a methyl group; and
R⁶ represents a carboxylic acid-protective group,
wherein a compound of a general formula: 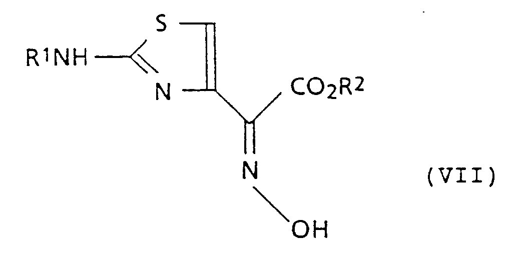 wherein R² represents a lower alkyl group and R¹ as the same meaning as mentioned above, is reacted with a compound of a general formula:
R³―O⁻M⁺ (VIII)
wherein R³ represents a lower alkyl group and M represents an alkali metal, in allyl alcohol to give a compound of a general formula: 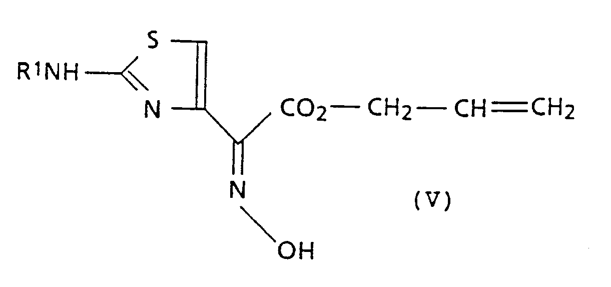 wherein R¹ has the same meaning as mentioned above, and thereafter the resulting compound is reacted with a compound of a general formula:

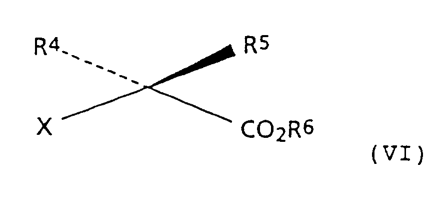

3. A method for preparing compounds of a general formula: 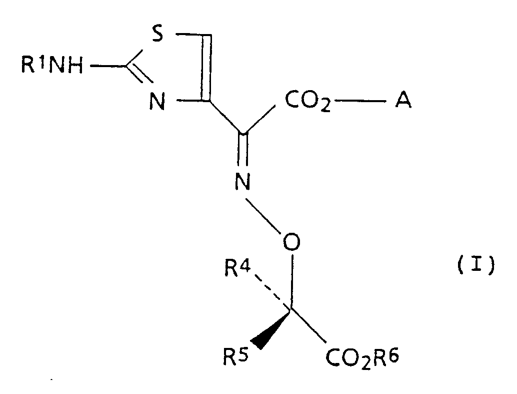 wherein R¹ represents an easily cleavable amino-protective group;
R⁴ and R⁵ are different from each other and each represents a hydrogen atom or a methyl group;
R⁶ represents a carboxylic acid-protective group; and
A represents a hydrogen atom, an alkali metal or a quaternary ammonium salt;
wherein a compound of a general formula: 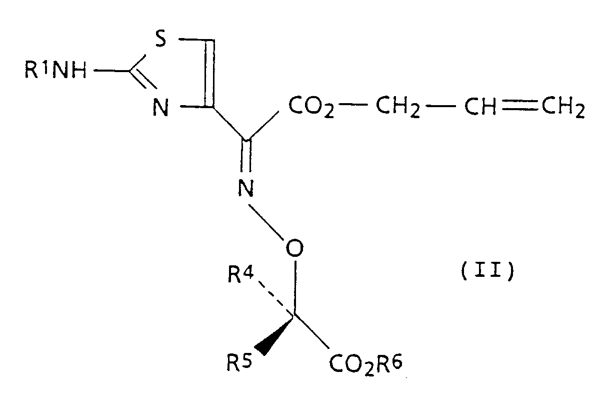 wherein R¹, R⁴, R⁵ and R⁶ have the same meanings as mentioned above, is reacted with a palladium salt or a palladium compound in the presence of a triphenylphosphine or a trialkyl phosphite and an alkali metal alkanoate or an organic base.

## Patentansprüche

1. Verfahren zur Herstellung von Verbindungen der allgemeinen Formel 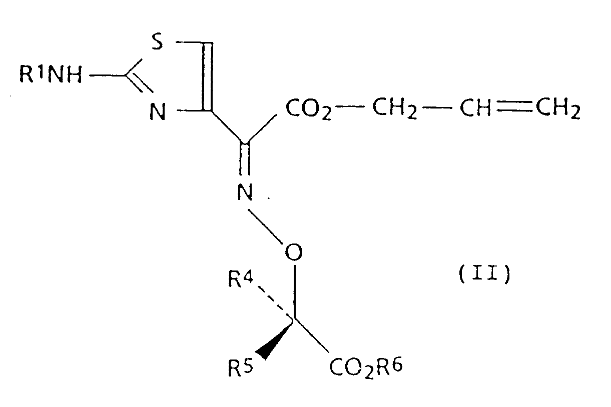 worin R¹ eine leicht abspaltbare Schutzgruppe für die Aminogruppe bedeutet;
R⁴ und R⁵ verschieden voneinander sind und jeweils ein Wasserstoffatom oder eine Methylgruppe bedeuten;
und R⁶ eine Schutzgruppe für die Carbonsäuregruppe bedeutet;
dadurch gekennzeichnet, daß man eine Verbindung der allgemeinen Formel 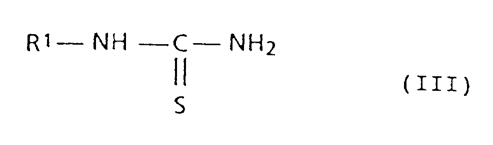 worin R¹ die oben angegebene Bedeutung hat, mit einer Verbindung der allgemeinen Formel 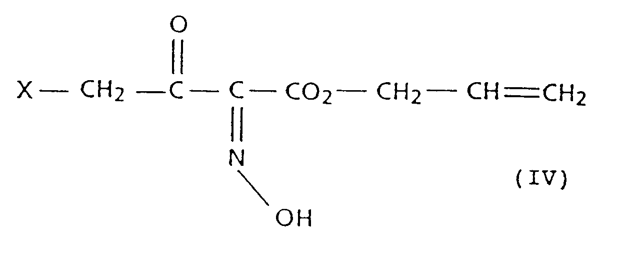 worin X eine abspaltbare Gruppe bedeutet unter Bildung einer Verbindung der allgemeinen Formel 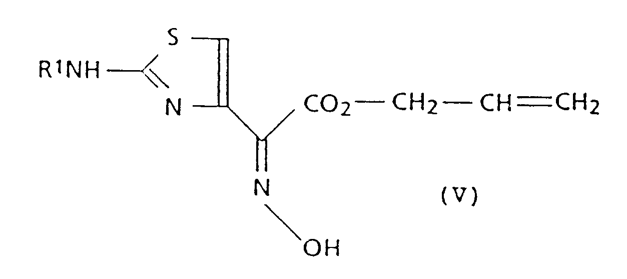 umsetzt, worin R¹ die oben angegebene Bedeutung hat, und danach die erhaltene Verbindung mit einer Verbindung der allgemeinen Formel 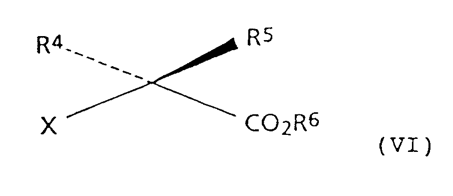 umsetzt, worin R⁴, R⁵, R⁶ und X die oben angegebenen Bedeutungen haben.

2. Verfahren zur Herstellung von Verbindungen der allgemeinen Formel 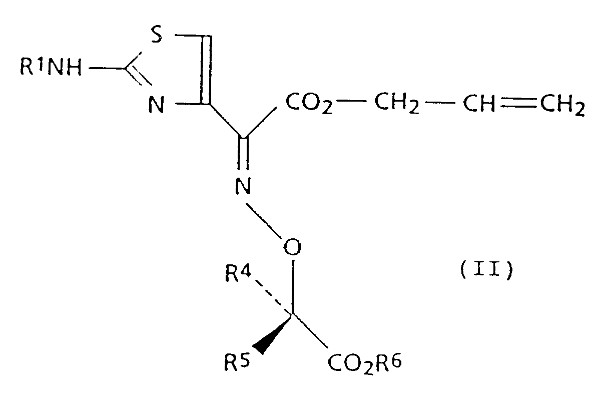 worin R¹ eine leicht abspaltbare Schutzgruppe für die Aminogruppe bedeutet;
R⁴ und R⁵ verschieden voneinander sind und jeweils ein Wasserstoffatom oder eine Methylgruppe bedeuten; und
R⁶ eine Schutzgruppe für eine Carbonsäuregruppe bedeutet,
dadurch gekennzeichnet, daß man eine Verbindung der allgemeinen Formel 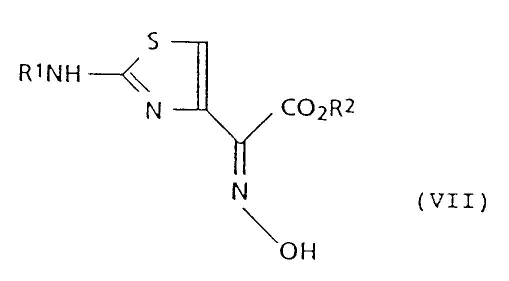 worin R² eine niedere Alkylgruppe bedeutet und R¹ die oben angegebene Bedeutung hat, mit einer Verbindung der allgemeinen Formel
R³―O⁻M⁺ (VIII)
in Allylakohol umsetzt, worin R³ eine niedere Alkylgruppe und M ein Alkalimetall bedeuten, unter Bildung einer Verbindung der allgemeinen Formel 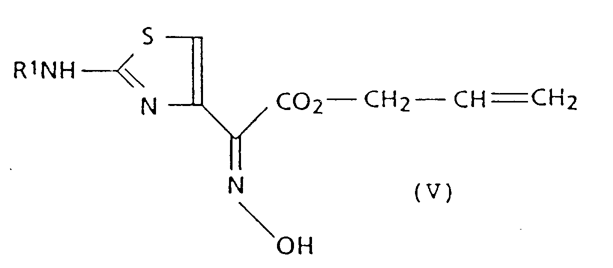 worin R¹ die oben angegebene Bedeutung hat und danach die erhaltene Verbindung mit einer Verbindung der allgemeinen Formel 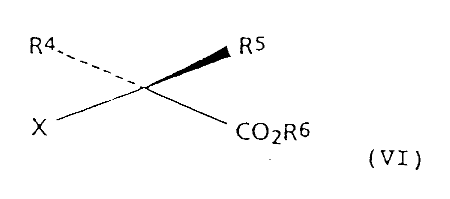 umsetzt.

3. Verfahren zur Herstellung von Verbindungen der allgemeinen Formel 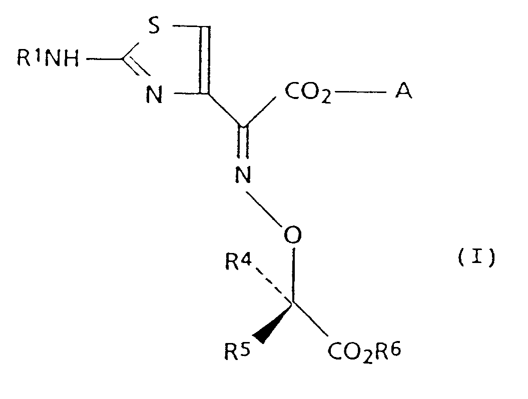 worin R¹ eine leicht abspaltbare Schutzgruppe für die Aminogruppe bedeutet,
R⁴ und R⁵ voneinander verschieden sind und jeweils ein Wasserstoffatom oder eine Methylgruppe bedeuten;
R⁶ eine Schutzgruppe für eine Carbonsäuregruppe bedeutet; und
A ein Wasserstoffatom, ein Alklimetall oder ein quaternäres Ammoniumsalz bedeutet,
dadurch gekennzeichnet, daß man eine Verbindung der allgemeinen Formel 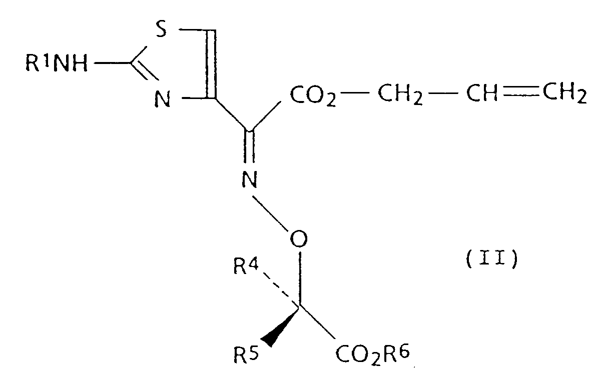 worin R¹, R⁴, R⁵ und R⁶ die oben angegebenen Bedeutungen haben, mit einem Palladiumsalz oder einer Palladiumverbindung in Gegenwart eines Triphenylphosphins oder eines Trialkylphosphits und eines Alkalialkanoats oder einer organischen Base umsetzt.

## Revendications

1. Procédé de préparation de composés répondant à la formule générale : 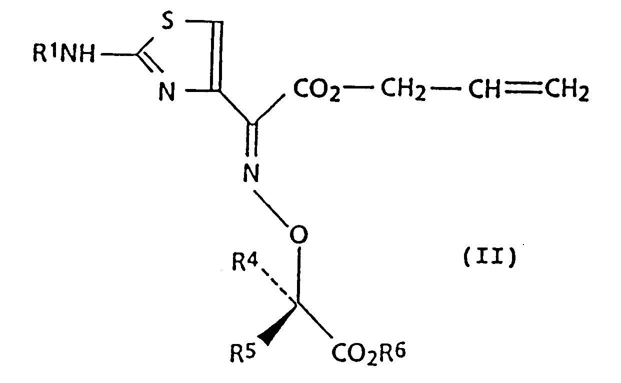 dans laquelle R¹ représente un groupe protecteur pour le groupe amino, qui peut être facilement enlevé par clivage ;
R⁴ et R⁵ sont différents l'un de l'autre et représentent chacun un atome d'hydrogène ou un groupe méthyle ; et
R⁶ représente un groupe protecteur pour l'acide carboxylique ;
dans lequel on fait réagir un composé répondant à formule générale : 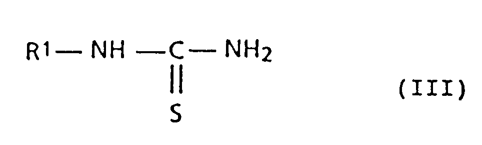 dans laquelle R¹ a la même signification que celle indiquée ci-dessus,
avec un composé répondant à la formule générale : 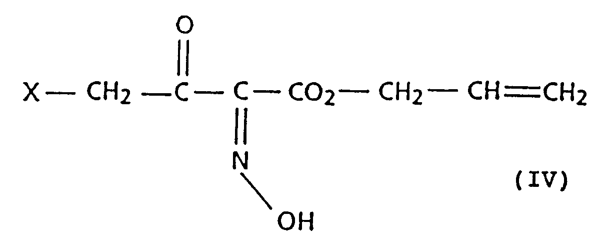 dans laquelle X représente un groupe labile,
pour obtenir un composé répondant à la formule générale : 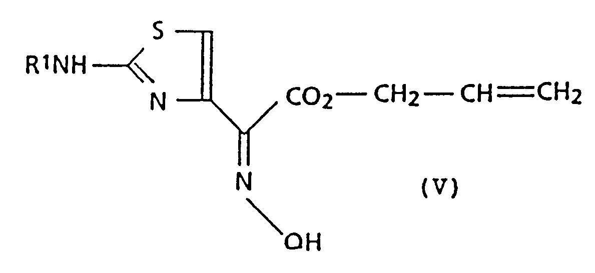 dans laquelle R¹ a la même signification que celle indiquée ci-dessus, et ensuite, on fait réagir le composé résultant avec un composé répondant à la formule générale : 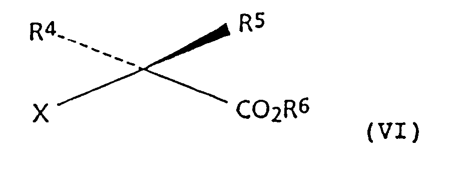 dans laquelle R⁴, R⁵, R⁶ et X ont les mêmes significations que celles indiquées ci-dessus.

2. Procédé de préparation de composés répondant à la formule générale : 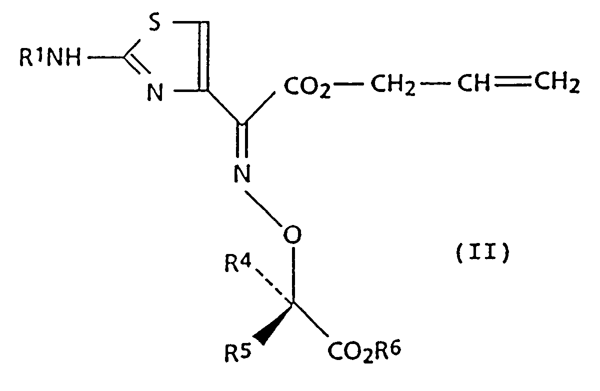 dans laquelle R¹ représente un groupe protecteur pour le groupe amino, qui peut être facilement enlevé par clivage ;
R⁴ et R⁵ sont différents l'un de l'autre et représentent chacun un atome d'hydrogène ou un groupe méthyle ; et
R⁶ représente un groupe protecteur pour l'acide carboxylique ;
dans lequel on fait réagir un composé répondant à la formule générale : 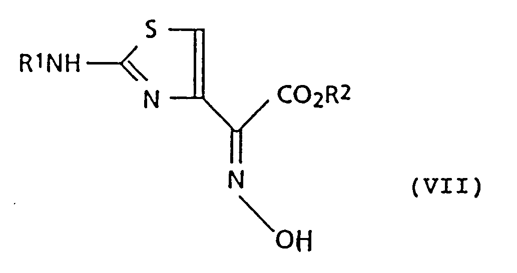 dans laquelle R² représente un groupe alkyle inférieur, et R¹ a la même signification que celle indiquée ci-dessus,
avec un composé répondant à la formule générale :
R³―O⁻M⁺ (VIII)
dans laquelle R³ représente un groupe alkyle inférieur, et M représente un métal alcalin,
dans de l'alcool allylique pour obtenir un composé répondant à la formule générale : 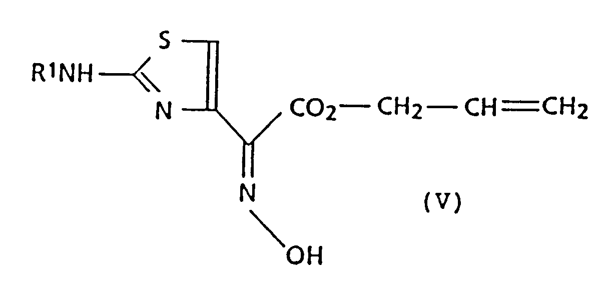 dans laquelle R¹ a la même signification que celle indiquée ci-dessus, et ensuite, on fait réagir le composé résultant avec un composé répondant à la formule générale :

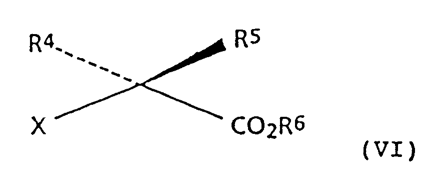

3. Procédé de préparation de composés répondant à la formule générale : 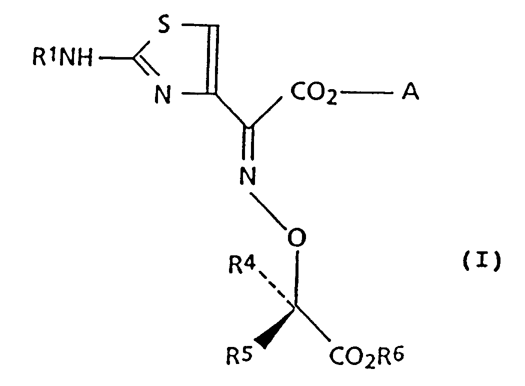 dans laquelle R¹ représente un groupe protecteur pour le groupe amino, qui peut être facilement enlevé par clivage ;
R⁴ et R⁵ sont différents l'un de l'autre et représentent chacun un atome d'hydrogène ou un groupe méthyle ;
R⁶ représente un groupe protecteur pour l'acide carboxylique ; et
A représente un atome d'halogène, un métal alcalin ou un sel d'ammonium quaternaire ;
dans lequel on fait réagir un composé répondant à la formule générale : 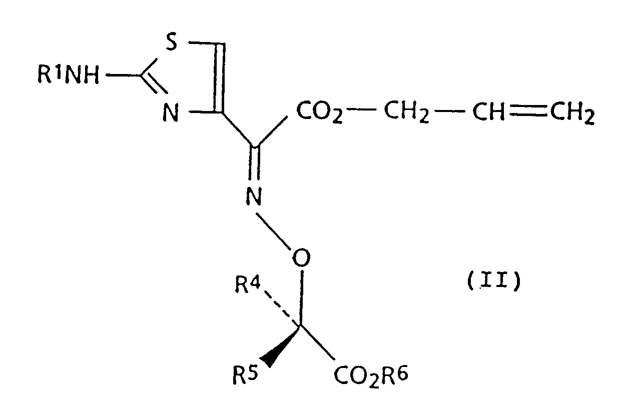 dans laquelle R¹, R⁴, R⁵ et R⁶ ont les mêmes significations que celles indiquées ci-dessus,
avec un sel de palladium ou un composé du palladium, en présence d'une triphénylphosphine ou d'un trialkylphosphite et d'un alcanoate de métal alcalin ou d'une base organique.
